# EUROPEAN PATENT APPLICATION

(11) **EP 3 026 037 A1**
(43) Date of publication of application: **01.06.2016**
(21) Application number: 14194883.6
(22) Date of filing: 26.11.2014
(51) Int. Cl.: C07C 5/48, C07C 51/25

(54) **Alkane oxidative dehydrogenation and/or alkene oxidation**

(71) Applicant: Shell Internationale Research Maatschappij B.V., 2596 HR Den Haag (NL)
(72) Inventor: Schoonebeek, Ronald Jan, 1031 HW Amsterdam (NL); Verhak, Michael Johannes Franciscus Maria, 1031 HW Amsterdam (NL)
(74) Representative: Matthezing, Robert Maarten

(57) **Abstract**

The invention relates to a process of the oxidative dehydrogenation of an alkane containing 2 to 6 carbon atoms and/or the oxidation of an alkene containing 2 to 6 carbon atoms, wherein a gas stream comprising oxygen and the alkane and/or alkene is fed to the inlet of a reactor comprising a reactor inlet, a catalyst bed and a reactor outlet and is contacted with a mixed metal oxide catalyst containing molybdenum, vanadium, niobium and optionally tellurium in the catalyst bed, and wherein the gas stream coming out of the reactor outlet comprises oxygen.

## Description

### Field of the invention

The present invention relates to a process of alkane oxidative dehydrogenation (oxydehydrogenation; ODH) and/or alkene oxidation.

### Background of the invention

It is known to oxidatively dehydrogenate alkanes, such as alkanes containing 2 to 6 carbon atoms, for example ethane or propane resulting in ethylene and propylene, respectively, in an oxidative dehydrogenation (oxydehydrogenation; ODH) process. Examples of alkane ODH processes, including catalysts and other process conditions, are for example disclosed in US7091377, WO2003064035, US20040147393, WO2010096909 and US20100256432. Mixed metal oxide catalysts containing molybdenum (Mo), vanadium (V), niobium (Nb) and optionally tellurium (Te) as the metals, can be used as such oxydehydrogenation catalysts. Such catalysts may also be used in the direct oxidation of alkenes to carboxylic acids, such as in the oxidation of alkenes containing 2 to 6 carbon atoms, for example ethylene or propylene resulting in acetic acid and acrylic acid, respectively.

It is an objective of the present invention to provide an alkane ODH and/or alkene oxidation process, wherein a mixed metal oxide catalyst containing Mo, V, Nb and optionally Te is used, which process is performed such that a relatively high activity and/or a relatively high selectivity is or are obtained. In general, a better catalyst performance is desired. More in particular, it is desired that during continuous operation, the catalyst life is extended and/or the catalyst activity and/or selectivity is or are maintained or even improved.

### Summary of the invention

Surprisingly it was found that one or more of the above-mentioned objectives can be obtained by means of an alkane ODH and/or alkene oxidation process, wherein the gas stream coming out of the reactor outlet comprises oxygen.

Accordingly, the present invention relates to a process of the oxidative dehydrogenation of an alkane containing 2 to 6 carbon atoms and/or the oxidation of an alkene containing 2 to 6 carbon atoms, wherein a gas stream comprising oxygen and the alkane and/or alkene is fed to the inlet of a reactor comprising a reactor inlet, a catalyst bed and a reactor outlet and is contacted with a mixed metal oxide catalyst containing molybdenum, vanadium, niobium and optionally tellurium in the catalyst bed, and wherein the gas stream coming out of the reactor outlet comprises oxygen.

### Detailed description of the invention

In the present invention, the gas stream coming out of the reactor outlet comprises oxygen, in a process wherein a gas stream comprising oxygen and an alkane and/or alkene is fed to the inlet of a reactor comprising a reactor inlet, a catalyst bed and a reactor outlet and is contacted with a mixed metal oxide catalyst containing molybdenum, vanadium, niobium and optionally tellurium in the catalyst bed. It has surprisingly been found that the presence of oxygen in said gas stream exiting the reactor, has a positive effect on both the catalyst activity and the catalyst selectivity during continuous operation. If no oxygen is present in said reactor outlet stream, it has appeared that both the activity and the selectivity of the above-mentioned catalyst are decreased permanently, even when the oxygen feed rate is increased again such that oxygen is present in said reactor outlet stream again. By controlling that oxygen is present in said outlet gas stream, it is ensured that in the entire reactor oxygen is present, especially near the outlet of the reactor where the amount of oxygen is reduced because of oxygen consumption in the alkane oxidative dehydrogenation (ODH) and/or alkene oxidation process, thereby apparently preventing the catalyst in the catalyst bed from getting a worse catalyst performance in terms of both activity and selectivity.

The presence of any amount of oxygen in the outlet gas stream can be controlled in any way. For example, at a certain constant conversion level, the amount of oxygen fed to the reactor may be increased to such extent that a certain amount of (unconverted) oxygen is present in said outlet gas stream. It is also possible to maintain the amount of the oxygen in the feed at a certain constant level and at the same time reduce the conversion level, for example by reducing the temperature, so that more (unconverted) oxygen is present in said outlet gas stream.

Preferably, in the present invention, the mixed metal oxide catalyst containing molybdenum, vanadium, niobium and optionally tellurium is a heterogeneous catalyst. Preferably, said heterogeneous catalyst is in the form of particles, in other words a particulate catalyst. Further, preferably, said heterogeneous catalyst is porous, specificly a porous, particulate catalyst. Inside the reactor, heterogeneous catalyst particles may make up the catalyst bed through which the gas stream comprising oxygen and the alkane and/or alkene is sent. In addition to catalyst particles, the catalyst bed may also contain inert (that is to say, catalytically inactive) particles.

Thus, in the present invention, the gas stream coming out of the reactor outlet comprises oxygen. The amount of oxygen in the gas stream coming out of the reactor outlet is not essential, in relation to the performance of the catalyst including its activity as discussed above, and may therefore vary within wide ranges. Preferably, the amount of oxygen in the gas stream coming out of the reactor outlet is at least 10 parts per million by volume (ppmv) based on the total volume of the gas stream, more preferably at least 20 ppmv, more preferably at least 30 ppmv, more preferably at least 40 ppmv, more preferably at least 50 ppmv, more preferably at least 70 ppmv, more preferably at least 100 ppmv, most preferably at least 200 ppmv. Further, preferably, the amount of oxygen in the gas stream coming out of the reactor outlet is at most 10 vol.% based on the total volume of the gas stream, more preferably at most 5 vol.%, more preferably at most 2 vol.%, more preferably at most 1 vol.% (10,000 ppmv), more preferably at most 5,000 ppmv, more preferably at most 3,000 ppmv, more preferably at most 1,000 ppmv, most preferably at most 500 ppmv. Therefore, suitably, the amount of oxygen in the gas stream coming out of the reactor outlet may be of from 10 ppmv to 10 vol.%, more suitably 20 ppmv to 2 vol.%, more suitably 30 ppmv to 10,000 ppmv (1 vol.%), most suitably 50 ppmv to 5,000 ppmv, based on the total volume of the gas stream. Generally, it is preferred to keep the amount of (unconverted) oxygen in the gas stream coming out of the reactor outlet as low as possible, without however decreasing the catalyst activity and/or selectivity, as discussed above.

Further, in the alkane oxidative dehydrogenation process and/or alkene oxidation process of the present invention, the temperature is preferably of from 300 to 500 °C, more preferably of from 310 to 450 °C, even more preferably of from 320 to 420 °C, most preferably of from 330 to 420 °C.

Preferably, said temperature is at least 300 °C, more preferably at least 310 °C, more preferably at least 320 °C, more preferably at least 330 °C, more preferably at least 340 °C, more preferably at least 345 °C, more preferably at least 350 °C, more preferably at least 355 °C, most preferably at least 360 °C.

Further, preferably, said temperature is at most 500 °C, more preferably at most 480 °C, more preferably at most 460 °C, more preferably at most 450 °C, more preferably at most 440 °C, more preferably at most 430 °C, more preferably at most 420 °C, more preferably at most 410 °C, most preferably at most 400 °C.

Still further, in the alkane oxidative dehydrogenation process and/or alkene oxidation process of the present invention, typical pressures are 0.1-20 bara (i.e. "bar absolute"). Further, in a preferred embodiment of the present invention, the pressure is of from 0.1 to 15 bara, more preferably of from 0.5 to 10 bara, most preferably of from 1 to 5 bara.

In the present invention, one gas stream comprising oxygen and the alkane and/or alkene may be fed to the reactor. Alternatively, two or more gas streams may be fed to the reactor, which gas streams form a combined gas stream inside the reactor. For example, one gas stream comprising oxygen and another gas stream comprising an alkane, such as ethane, may be fed to the reactor separately. Said one gas stream or multiple gas streams may additionally comprise an inert gas, as further described below.

Preferably, in the alkane oxidative dehydrogenation process of the present invention, the alkane containing 2 to 6 carbon atoms is a linear alkane in which case said alkane may be selected from the group consisting of ethane, propane, butane, pentane and hexane. Further, preferably, said alkane contains 2 to 4 carbon atoms and is selected from the group consisting of ethane, propane and butane. More preferably, said alkane is ethane or propane. Most preferably, said alkane is ethane.

Further, preferably, in the alkene oxidation process of the present invention, the alkene containing 2 to 6 carbon atoms is a linear alkene in which case said alkene may be selected from the group consisting of ethylene, propylene, butene, pentene and hexene. Further, preferably, said alkene contains 2 to 4 carbon atoms and is selected from the group consisting of ethylene, propylene and butene. More preferably, said alkene is ethylene or propylene.

The product of said alkane oxidative dehydrogenation process may comprise the dehydrogenated equivalent of the alkane, that is to say the corresponding alkene. For example, in the case of ethane such product may comprise ethylene, in the case of propane such product may comprise propylene, and so on. Such dehydrogenated equivalent of the alkane is initially formed in said alkane oxidative dehydrogenation process. However, in said same process, said dehydrogenated equivalent may be further oxidized under the same conditions into the corresponding carboxylic acid which may or may not contain one or more unsaturated double carbon-carbon bonds. As mentioned above, it is preferred that the alkane containing 2 to 6 carbon atoms is ethane or propane. In the case of ethane, the product of said alkane oxidative dehydrogenation process may comprise ethylene and/or acetic acid, preferably ethylene. Further, in the case of propane, the product of said alkane oxidative dehydrogenation process may comprise propylene and/or acrylic acid, preferably acrylic acid.

The product of said alkene oxidation process comprises the oxidized equivalent of the alkene. Preferably, said oxidized equivalent of the alkene is the corresponding carboxylic acid. Said carboxylic acid may or may not contain one or more unsaturated double carbon-carbon bonds. As mentioned above, it is preferred that the alkene containing 2 to 6 carbon atoms is ethylene or propylene. In the case of ethylene, the product of said alkene oxidation process may comprise acetic acid. Further, in the case of propylene, the product of said alkene oxidation process may comprise acrylic acid.

The present alkane oxidative dehydrogenation process and/or alkene oxidation process comprises contacting a gas stream comprising oxygen (O₂) and the alkane and/or alkene with the mixed metal oxide catalyst. Said gas stream may be a combination of 2 gas streams being fed separately to the reator, one gas stream comprising the oxygen and one gas stream comprising the alkane and/or alkene. Additionally, said gas stream comprising oxygen and the alkane and/or alkene may contain an inert gas. Said inert gas may be selected from the group consisting of the noble gases and nitrogen (N₂). Preferably, the inert gas is nitrogen or argon, more preferably nitrogen. Said oxygen (O₂) is an oxidizing agent, thereby resulting in oxidative dehydrogenation of the alkane and/or oxidation of the alkene. Said oxygen may originate from any source, such as for example air.

Ranges for the molar ratio of oxygen to the alkane and/or alkene in said gas stream which are suitable, are of from 0.01 to 1, more suitably 0.05 to 0.5. Furthermore, in a preferred embodiment, said gas stream comprises 5 to 35 vol.% of oxygen, more suitably 20 to 30 vol.% of oxygen, and 40 to 80 vol.% of the alkane and/or alkene, more suitably 50 to 70 vol.% of the alkane and/or alkene, and less than 80 (0 to 80) vol.% of the above-mentioned inert gas, more suitably less than 50 (0 to 50) vol.% of said inert gas, more suitably 5 to 35 vol.% of said inert gas, most suitably 10 to 20 vol.% of said inert gas. In the context of the present invention, the components of said gas stream are to be selected in an overall amount not to exceed 100 vol.%.

Said ratio of oxygen to the alkane and/or alkene and said volume percentages for oxygen, the alkane and/or alkene and said inert gas are the ratio and volume percentages, respectively, at the entrance of the catalyst bed. Obviously, after entering the catalyst bed, at least part of the oxygen and alkane and/or alkene from the gas stream gets consumed.

In the present invention, the catalyst is a mixed metal oxide catalyst containing molybdenum, vanadium, niobium and optionally tellurium as the metals, which catalyst may have the following formula:

Mo₁VₐTe_{b}Nb_{c}Oₙ

wherein:
a, b, c and n represent the ratio of the molar amount of the element in question to the molar amount of molybdenum (Mo);
a (for V) is from 0.01 to 1, preferably 0.05 to 0.60, more preferably 0.10 to 0.40, more preferably 0.20 to 0.35, most preferably 0.25 to 0.30;
b (for Te) is 0 or from >0 to 1, preferably 0.01 to 0.40, more preferably 0.05 to 0.30, more preferably 0.05 to 0.20, most preferably 0.09 to 0.15;
c (for Nb) is from >0 to 1, preferably 0.01 to 0.40, more preferably 0.05 to 0.30, more preferably 0.10 to 0.25, most preferably 0.14 to 0.20; and
n (for O) is a number which is determined by the valency and frequency of elements other than oxygen.

Examples of oxydehydrogenation processes, including catalysts and other process conditions, are for example disclosed in above-mentioned US7091377, WO2003064035, US20040147393, WO2010096909 and US20100256432, the disclosures of which are herein incorporated by reference.

The amount of the catalyst in said process is not essential. Preferably, a catalytically effective amount of the catalyst is used, that is to say an amount sufficient to promote the alkane oxydehydrogenation and/or alkene oxidation reaction.

In general, the product stream comprises water in addition to the desired product. Water may easily be separated from said product stream, for example by cooling down the product stream from the reaction temperature to a lower temperature, for example room temperature, so that the water condenses and can then be separated from the product stream.

The invention is further illustrated by the following Examples.

### Examples

### (A) Preparation of the catalyst

A mixed metal oxide catalyst containing molybdenum (Mo), vanadium (V), niobium (Nb) and tellurium (Te) was prepared, for which catalyst the molar ratio of said 4 metals was Mo₁V_{0.29}Nb_{0.17}Te_{0.12}.

Two solutions were prepared. Solution 1 was obtained by dissolving 15.8 g of ammonium niobate oxalate and 4.0 g of oxalic acid dihydrate in 160 ml of water at room temperature. Solution 2 was prepared by dissolving 35.6 g of ammonium heptamolybdate, 6.9 g of ammonium metavanadate and 5.8 g of telluric acid (Te(OH)₆) in 200 ml of water at 70 °C. 7.0 g of concentrated nitric acid was then added to solution 2. The 2 solutions were combined which yielded an orange gel-like precipitate. The mixture was evaporated to dryness with the aid of a rotating evaporator ("rotavap") at 50 °C.

The dried material was further dried in static air at 120 °C for 16 hours, milled to a fine powder and then calcined in static air at a temperature of 325 °C for 2 hours. After the air calcination, the material was further calcined in a nitrogen (N₂) stream at 600 °C for 2 hours. Then the material was treated with an aqueous 5% oxalic acid solution at 80 °C and filtered and dried at 120 °C.

The dried catalyst powder was pressed into pills which pills were then milled. The milled material was then sieved using a sieve having a mesh size of 40-80 mesh. The sieved material, having a size of 40-80 mesh and composed of porous catalyst particles, was then used in the ethane oxidative dehydrogenation experiments described below.

### (B) Catalytic oxidative dehydrogenation of ethane

The catalyst thus prepared was used in experiments involving ethane oxidative dehydrogenation (ethane ODH) within a small-scale testing unit comprising a vertically oriented, cylindrical, quartz reactor having an inner diameter of 3.0 mm. 0.65 g of the catalyst was loaded in the reactor. The catalyst bed height was 6 cm. On top of the catalyst bed, another bed having a height of 8 cm was placed which latter bed contained inert silicon carbide (SiC) particles having an average diameter of 0.8 mm.

In these experiments, a gas stream comprising 63 vol.% of ethane, 21 vol.% of oxygen (O₂) and 16 vol.% of nitrogen (N₂) was fed to the top of the reactor and then sent downwardly through the catalyst bed to the bottom of the reactor. Said gas stream was a combined gas stream comprising a flow of ethane having a rate of 3.00 Nl/hr, a flow of oxygen having a rate of 1.00 Nl/hr and a flow of nitrogen having a rate of 0.77 Nl/hr. "N1" stands for "normal litre" as measured at standard temperature and pressure, namely 32 °F (0 °C) and 1 bara (100 kPa). The pressure in the reactor was 2.3 bara. The reactor was heated such that the temperature of the catalyst (at the end of the catalyst bed) was 370 °C.

The conversion of ethane and the product composition were measured with a gas chromatograph (GC) equipped with a thermal conductivity detector (TCD) and with another GC equipped with a flame ionization detector. Acetic acid byproduct and water from the reaction were trapped in a quench pot.

The above conditions (hereinafter referred to as "reference conditions") were maintained for 100 hours (Period A). Under these conditions, the oxygen conversion was not complete and the gas stream coming out of the reactor outlet comprised (unconverted) oxygen in an amount of 3.85 vol.%, based on the total volume of the gas stream.

Then the oxygen flow rate was decreased from 1.00 to 0.60 Nl/hr. Further, the nitrogen flow rate was increased from 0.77 to 1.17 Nl/hr, so that the total flow rate was not changed. Under these conditions, the oxygen conversion was complete and the gas stream coming out of the reactor outlet did not comprise oxygen.

The latter conditions were maintained for 60 hours (Period B), and then the above-mentioned reference conditions were restored and maintained for 25 hours (Period C).

In Table 1 below, the experimental results (conversion of ethane and selectivity towards ethylene) for above-mentioned Periods A and C are shown.

**Table 1**

| Period | Conversion of ethane (%) | Selectivity to ethylene (%) |
|---|---|---|
| A | 41.3 | 92.9 |
| C | 37.1 | 90.9 |

Surprisingly, it appears that after a period wherein the gas stream coming out of the reactor outlet comprised no oxygen (above-mentioned Period B), in which case oxygen is not present in the entire reactor, especially near the outlet of the reactor where no oxygen is present because of complete oxygen consumption, the conversion and selectivity drop significantly in a subsequent period wherein the gas stream coming out of the reactor outlet does comprise oxygen again by increasing the oxygen flow rate to its original level (above-mentioned Period C), as compared to the period before the oxygen flow rate was decreased (above-mentioned Period A).

## Claims

1. Process of the oxidative dehydrogenation of an alkane containing 2 to 6 carbon atoms and/or the oxidation of an alkene containing 2 to 6 carbon atoms, wherein a gas stream comprising oxygen and the alkane and/or alkene is fed to the inlet of a reactor comprising a reactor inlet, a catalyst bed and a reactor outlet and is contacted with a mixed metal oxide catalyst containing molybdenum, vanadium, niobium and optionally tellurium in the catalyst bed, and wherein the gas stream coming out of the reactor outlet comprises oxygen.

2. Process according to claim 1, wherein the amount of oxygen in the gas stream coming out of the reactor outlet is at least 10 parts per million by volume (ppmv), based on the total volume of the gas stream

3. Process according to claim 1 or 2, wherein the amount of oxygen in the gas stream coming out of the reactor outlet is of from 10 ppmv to 10 vol.%, more suitably 30 ppmv to 10,000 ppmv, most suitably 50 ppmv to 5,000 ppmv, based on the total volume of the gas stream.

4. Process according to any one of the preceding claims, wherein the temperature is of from 300 to 500 °C, preferably of from 310 to 450 °C, more preferably of from 320 to 420 °C, most preferably of from 330 to 420 °C.

5. Process according to any one of the preceding claims, wherein the pressure is of from 0.1 to 15 bara, preferably of from 0.5 to 10 bara, more preferably of from 1 to 5 bara.

6. Process according to any one of the preceding claims, wherein the process is a process of the oxidative dehydrogenation of an alkane containing 2 to 6 carbon atoms and wherein said alkane is ethane or propane, preferably ethane.

7. Process according to any one of claims 1 to 5, wherein the process is a process of the oxidation of an alkene containing 2 to 6 carbon atoms and wherein said alkene is ethylene or propylene.
